# EUROPEAN PATENT APPLICATION

(11) **EP 2 735 613 A1**
(43) Date of publication of application: **28.05.2014**
(21) Application number: 12818038.7
(22) Date of filing: 17.07.2012
(51) Int. Cl.: C12N 15/09, A01K 67/027, A61K 31/7105, A61K 31/713, A61K 48/00, A61P 25/00, A61P 35/00, A61P 37/08, C12N 5/10

(54) **TECHNIQUE FOR CLEAVING OUT PART OF poly(A) CHAIN AND/OR 3'-TERMINAL SEQUENCE OF mRNA TO INHIBIT TRANSLATION REACTION**

(30) Priority: 22.07.2011 JP 2011160512
(71) Applicant: Public University Corporation Yokohama City University, Yokohama-shi, Kanagawa 236-0027 (JP); Yoshindo Inc., Toyama 939-2723 (JP); Tokyo Institute of Technology, Tokyo 152-8550 (JP)
(72) Inventor: WADA, Tadashi, Yokohama-shi Kanagawa 230-0045 (JP); TAKEDA, Kei, Toyama-shi Toyama 939-2723 (JP); HANDA, Hiroshi, Tokyo 152-8550 (JP)
(74) Representative: Ford, Hazel
(86) International application number: PCT/JP2012/068085
(87) International publication number: WO 2013/015152

(57) **Abstract**

The present invention provides a method of artificially repressing gene expression, which is simpler to design than conventional methods (the RNAi, ribozyme and antisense methods) and which allows for easier confirmation of the effect.

A method of inhibiting the translation reaction of a target gene, comprising cutting out a part of the poly(A) tail and/or 3'-terminal sequence of the target mRNA is provided. Also provided are a kit for inhibiting the translation reaction of a target gene, comprising a reagent capable of cutting out a part of the poly(A) tail and/or 3'-terminal sequence of the target mRNA; a cell in which the translation reaction of a target gene is inhibited by introduction thereinto of a reagent capable of cutting out a part of the poly(A) tail and/or 3'-terminal sequence of the target mRNA; and a non-human organism in which the translation reaction of a target gene is inhibited by introduction thereinto of a reagent capable of cutting out a part of the poly(A) tail and/or 3'-terminal sequence of the target mRNA.

SEQUENCE LISTING

<110>  Public University Corporation Yokohama City University
       Yoshindo Inc.
       Tokyo Institute of Technology

<120>  Technique for inhibiting translation by cleaving a part of polyA tail
and/or 3′ end sequence of mRNA

<130>  FP-172PCT

<150>  JP P2011-160512
<151>  2011-07-22

<160>  43

<170>  PatentIn version 3.1

<210>  1
<211>  25
<212>  DNA
<213>  Artificial

<220>
<223>  cdk9 MO

<400>  1
ggaaatgtga aggatttata ggtgt                                          25

<210>  2
<211>  25
<212>  DNA
<213>  Artificial

<220>
<223>  cdk9 MO-2

<400>  2
atttatactt atacaagtaa caaac                                          25

<210>  3
<211>  25
<212>  DNA
<213>  Artificial

<220>
<223>  cdk9 MO-3

<400>  3
accatgaccc cgaacacgtg atctt                                          25

<210>  4
<211>  25
<212>  DNA
<213>  Artificial

<220>
<223>  cdk9 MO-4

<400>  4
acaaataaaa acatctttaa aaata                                          25

<210>  5
<211>  25
<212>  DNA
<213>  Artificial

<220>
<223>  cdk9 MO-5

<400>  5
tgtgaaggat ttattggtgt attta                                          25


<210>  6
<211>  25
<212>  DNA
<213>  Artificial

<220>
<223>  cdk9 MO-6

<400>  6
aggatttatt ggtgtattta tactt                                          25


<210>  7
<211>  25
<212>  DNA
<213>  Artificial

<220>
<223>  cdk9 MO-7

<400>  7
ttattggtgt atttatactt ataca                                          25


<210>  8
<211>  25
<212>  DNA
<213>  Artificial

<220>
<223>  cdk9 MO-8

<400>  8
ggtgtattta tacttataca agtaa                                          25


<210>  9
<211>  25
<212>  DNA
<213>  Artificial

<220>
<223>  cdk9m MO

<400>  9
ggtaatatga acgatgtata ggtgt                                          25


<210>  10
<211>  30
<212>  DNA

<213>  Artificial

<220>
<223>  (dT)12-anchor primer

<400>  10
gcgagctccg cggccgcgtt tttttttttt                                        30


<210>  11
<211>  20
<212>  DNA
<213>  Artificial

<220>
<223>  zcdk9 PAT primer

<400>  11
gtgctgcccc agtgcattgt                                                   20


<210>  12
<211>  21
<212>  DNA
<213>  Artificial

<220>
<223>  tbp PAT primer

<400>  12
tgttgtgcag tgcgagagat c                                                 21


<210>  13
<211>  21
<212>  DNA
<213>  Artificial

<220>
<223>  cyclin B1 PAT primer

<400>  13
atgttgtgag ggtcaacgag g                                                 21


<210>  14
<211>  22
<212>  DNA
<213>  Artificial

<220>
<223>  cyclin B2 PAT primer

<400>  14
agcagcagac tcatgaagat ca                                                22


<210>  15
<211>  20
<212>  DNA
<213>  Artificial

<220>
<223>  actin-specific primer (forward)

<400>  15
ctgaatccca aagccaacag                                                   20


<210>  16
<211>  20
<212>  DNA
<213>  Artificial

<220>
<223>  actin-specific primer (reverse)

<400>  16
tcacaccatc accagagtcc                                                   20


<210>  17
<211>  20
<212>  DNA
<213>  Artificial

<220>
<223>  biklf-specific primer (forward)

<400>  17
atgctgactc caccatcctc                                                   20


<210>  18
<211>  20
<212>  DNA
<213>  Artificial

<220>
<223>  biklf-specific primer (reverse)

<400>  18
tgtccggtgt gtttcctgta                                                   20


<210>  19
<211>  20
<212>  DNA
<213>  Artificial

<220>
<223>  zcdk9-specific primer (forward)

<400>  19
cagccaatca gagttcgaca                                                   20


<210>  20
<211>  20
<212>  DNA
<213>  Artificial

<220>
<223>  zcdk9-specific primer (reverse)

<400>  20
tagtgccacc ggtaaactcc                                                   20


<210>  21

<211>  20
<212>  DNA
<213>  Artificial

<220>
<223>  tbp-specific primer (forward)

<400>  21
cttggggtgc aaacttgatt                                                    20


<210>  22
<211>  20
<212>  DNA
<213>  Artificial

<220>
<223>  tbp-specific primer (reverse)

<400>  22
catatttcct ggctgccaat                                                    20


<210>  23
<211>  20
<212>  DNA
<213>  Artificial

<220>
<223>  cyclin B1-specific primer (forward)

<400>  23
cagctgcaac ttgttggtgt                                                    20


<210>  24
<211>  20
<212>  DNA
<213>  Artificial

<220>
<223>  cyclin B1-specific primer (reverse)

<400>  24
ggtagaggcc ttccaaaacc                                                    20


<210>  25
<211>  20
<212>  DNA
<213>  Artificial

<220>
<223>  cyclin B2-specific primer (forward)

<400>  25
ctcaaagcat ctgacggtga                                                    20


<210>  26
<211>  20
<212>  DNA
<213>  Artificial

<220>

<223>  cyclin B2-specific primer (reverse)

<400>  26
gcagcagtcc atctctcaca                                                    20


<210>  27
<211>  19
<212>  RNA
<213>  Artificial

<220>
<223>  siRNA to RelA

<400>  27
cugaacuaau aaaucuguu                                                     19


<210>  28
<211>  19
<212>  RNA
<213>  Artificial

<220>
<223>  siRNA to Bcl-xL

<400>  28
guucaguaau aaacugugu                                                     19


<210>  29
<211>  19
<212>  RNA
<213>  Artificial

<220>
<223>  siRNA to Livin

<400>  29
gaauagaaau aaagugggu                                                     19


<210>  30
<211>  19
<212>  RNA
<213>  Artificial

<220>
<223>  siRNA to PLK1

<400>  30
uaugcacauu aaacagaug                                                     19


<210>  31
<211>  19
<212>  RNA
<213>  Artificial

<220>
<223>  siRNA to tPA

<400>  31
cuguacuuaa uaaauucag                                                     19

<210>  32
<211>  20
<212>  DNA
<213>  Artificial

<220>
<223>  RelA-specific primer (forward)

<400>  32
cctggagcag gctatcagtc                                                    20


<210>  33
<211>  20
<212>  DNA
<213>  Artificial

<220>
<223>  RelA-specific primer (reverse)

<400>  33
atcttgagct cggcagtgtt                                                    20


<210>  34
<211>  20
<212>  DNA
<213>  Artificial

<220>
<223>  Bcl-xL-specific primer (forward)

<400>  34
ggtattggtg agtcggatcg                                                    20


<210>  35
<211>  20
<212>  DNA
<213>  Artificial

<220>
<223>  Bcl-xL-specific primer (reverse)

<400>  35
aagagtgagc ccagcagaac                                                    20


<210>  36
<211>  20
<212>  DNA
<213>  Artificial

<220>
<223>  PLK1-specific primer (forward)

<400>  36
ggcaaccttt tcctgaatga                                                    20


<210>  37
<211>  20
<212>  DNA
<213>  Artificial

<220>
<223>  PLK1-specific primer (reverse)

<400>  37
aatggaccac acatccacct                                                   20


<210>  38
<211>  20
<212>  DNA
<213>  Artificial

<220>
<223>  tPA-specific primer (forward)

<400>  38
cccagatcga gactcaaagc                                                   20


<210>  39
<211>  20
<212>  DNA
<213>  Artificial

<220>
<223>  tPA-specific primer (reverse)

<400>  39
tggggttctg tgctgtgtaa                                                   20


<210>  40
<211>  20
<212>  DNA
<213>  Artificial

<220>
<223>  Livin-specific primer (forward)

<400>  40
cctctctgcc tgttctggac                                                   20


<210>  41
<211>  20
<212>  DNA
<213>  Artificial

<220>
<223>  Livin-specific primer (reverse)

<400>  41
ctccagggaa aacccacttt                                                   20


<210>  42
<211>  19
<212>  DNA
<213>  Artificial

<220>
<223>  bActin-specific primer (forward)

<400>  42

gatatcgccg cgctcgtcg                                                    19

<210>  43
<211>  18
<212>  DNA
<213>  Artificial

<220>
<223>  bActin-specific primer (reverse)

<400>  43
gggaggagct ggaagcag                                                     18

## Description

### TECHNICAL FIELD

The present invention relates to a technique for inhibiting the translation reaction of a target gene by cutting out a part of the poly(A) tail and/or 3'-terminal sequence of the target mRNA.

### BACKGROUND ART

As methods for artificially inhibiting gene expression, the RNAi method (Patent Document No. 1; Non-Patent Document No. 1), the ribozyme method (Patent Documents Nos. 2 and 3; Non-Patent Document No. 2) and the antisense method (Patent Document Nos. 4 and 5; Non-Patent Document No. 3) are known. The RNAi method and the ribozyme method inhibit gene expression by inducing specific degradation of the target mRNA with a nucleic acid or the like that specifically binds to the target mRNA. The antisense method inhibits gene expression by allowing duplex formation between the target mRNA and a nucleic acid or the like that specifically binds to the target mRNA, to thereby induce inhibition of translation reaction or normal splicing reaction.

The RNAi method and the ribozyme method are methods depending on an activity that induces cleavage of mRNA through a specific sequence (target sequence) consisting of several ten nucleotides within the target mRNA. Since the length of mRNA is usually 1000 nucleotides or more, a target sequence that will produce the effect of interest must be selected. For this purpose, software etc. are available but they are not practical and, instead, preliminary experiments are performed targeting a plurality of sites and then a target sequence that will produce the effect of interest is selected from them.

On the other hand, in the antisense method, target sequences are limited to two sites that are around the translation initiation codon and the splicing junction site. However, with the former site, the antisense effect can not be detected at the mRNA level, which makes the process of confirmation of the resultant effect complicated. In contrast, with the latter site, the antisense effect can be detected at the mRNA level but it is difficult to judge whether the resultant protein has lost its function or not.

### PRIOR ART LITERATURE

### Patent Documents

Patent Document No. 1: Japanese Unexamined Patent Publication No. 2009-291209
Patent Document No. 2: WO92/03456
Patent Document No. 3: Japanese Patent No. 2708960
Patent Document No. 4: WO88/04300A1
Patent Document No. 5: Japanese Patent No. 2530906

### Non-Patent Documents

Non-Patent Document No. 1: Nature. 2009 Jan 22;457(7228):396-404.
On the road to reading the RNA-interference code, Siomi H, Siomi MC.
Non-Patent Document No. 2: Chem Senses. 1998 Apr;23(2):249-55.
Current status of antisense DNA methods in behavioral studies.
Ogawa S, Pfaff DW
Non-Patent Document No. 3: Trends Genet. 1996 Dec;12(12):510-5.
Anti-gene therapy: the use of ribozymes to inhibit gene function.
Couture LA, Stinchcomb DT.

### DISCLOSURE OF THE INVENTION

### PROBLEM FOR SOLUTION BY THE INVENTION

It is an object of the present invention to provide a method of artificially inhibiting gene expression, which is simpler to design than conventional methods (the RNAi, ribozyme and antisense methods) and which allows for easier confirmation of the effect.

### MEANS TO SOLVETHE PROBLEM

The inventors have found that by selectively deleting a part of the poly(A) tail and/or 3'-terminal sequence of a target mRNA, translation reaction can be inhibited to thereby achieve a specific gene downregulating effect. The present invention has been achieved based on these findings.

A summary of the present invention is as described below.
(1) A method of inhibiting the translation reaction of a target gene, comprising cutting out a part of the poly(A) tail and/or 3'-terminal sequence of the target mRNA.
(2) The method of (1) above, wherein a part of the poly(A) tail and/or 3'-terminal sequence of the target mRNA is cut out with a ribonucleic acid which is capable of hybridizing to the part of the poly(A) tail and/or 3'-terminal sequence of the target mRNA.
(3) The method of (2) above, wherein the ribonucleic acid comprises a nucleotide sequence complementary to the whole or a part of a sequence encoding the region from the poly(A) tail junction of the target mRNA to the 40^{th} nucleotide upstream therefrom.
(4) The method of (3) above, wherein the whole or a part of a sequence encoding the region of 40 nucleotides from the poly(A) tail junction of the target mRNA to the 40^{th} nucleotide upstream therefrom contains the whole or a part of a polyadenylation signal sequence.
(5) The method of (3) or (4) above, wherein the ribonucleic acid is a 20- to 25-mer.
(6) The method of any one of (2) to (5) above, wherein the ribonucleic acid comprises natural nucleotides.
(7) The method of (6) above, wherein the ribonucleic acid is a double-stranded RNA.
(8) The method of any one of (2) to (5) above, wherein the ribonucleic acid contains at least one nucleotide analogue.
(9) The method of (8) above, wherein the ribonucleic acid is single-stranded.
(10) The method of (9), wherein the single-stranded ribonucleic acid is an antisense morpholino oligonucleotide.
(11) A kit for inhibiting the translation reaction of a target gene, comprising a reagent capable of cutting out a part of the poly(A) tail and/or 3'-terminal sequence of the target mRNA.
(12) The kit of (11) above, wherein the reagent capable of cutting out a part of the poly(A) tail and/or 3'-terminal sequence of the target mRNA is a ribonucleic acid.
(13) A cell in which the translation reaction of a target gene is inhibited by introduction thereinto of a reagent capable of cutting out a part of the poly(A) tail and/or 3'-terminal sequence of the target mRNA.
(14) A non-human organism in which the translation reaction of a target gene is inhibited by introduction thereinto of a reagent capable of cutting out a part of the poly(A) tail and/or 3'-terminal sequence of the target mRNA.

### EFFECT OF THE INVENTION

According to the present invention, gene downregulation has become possible that allows for simple determination of target sequences. Further, the present invention enables the effect of gene downregulation to be assessed at the mRNA level, thereby leading to easy assessment of the effect.

Further, according to the present invention, the translation reactions of a plurality of mRNAs generated from the same gene by selecttive splicing can be inhibited either simultaneously or selectively.

The present specification encompasses the contents disclosed in the specification and/or drawings of Japanese Patent Application No. 2011-160512 based on which the present application claims priority

### BRIEF DESCRIPTION OF THE DRAWINGS

[Fig. 1]
   Injection of cdk9 MO (morpholino oligonucleotide) into embryos inhibits poly(A) tail elongation and translation of *zcdk9* mRNA during early development, but injection of cdk9m MO does not cause such inhibition. (A) Target sequence within the *zcdk9* mRNA 3'-UTR to which MO hybridizes. (B) Inhibition of *zcdk9* poly(A) tail elongation in cdk9 MO-injected embryos. Embryos were collected at the times indicated. Total RNA was extracted from untreated (WT), cdk9 MO-injected and cdk9m MO-injected embryos. PAT assay was performed with PAT primers for *cdk9, tbp, cyclin B1,* and *cyclin B2.* PCR products were analyzed by 2.2% agarose gel electrophoresis. Right side: length markers in bases. (C) Specific translation inhibition *of zcdk9* mRNA with cdk9 MO. This inhibition does not occur with cdk9 MO. Embryos were collected at the times indicated. Western blotting was performed with extracts from untreated (lanes 1, 4, 7 and 10), cdk9 MO-injected (lanes 2, 5, 8 and 11) and cdk9m MO-injected embryos (lanes 3, 6, 9 and 12) by using the antibodies indicated. HeLa cell nuclear extract (NE) (lane 13) served as a control. (D) The cdk9 MO-and cdk9m MO-injected embryos were collected at 3, 4 and 5 hpf (hours post fertilization). Extracts from these embryos (as used in (C)) were analyzed by western blotting. (E and F) Reverse transcription using random primers (E) or oligo-dT primers (F), followed by PCR with primer sets for *cdk9, biklf, tbp* and *actin.* PCR products were analyzed by 2.2% agarose gel electrophoresis. Embryos were collected at the times indicated. Total RNA was extracted from untreated (WT: lanes 1 to 3), cdk9 MO-injected (cdk9 MO: lanes 4 to 6) and cdk9m MO-injected embryos (cdk9m MO: lanes 7 to 9).
[Fig. 2]
   The 40 nucleotides from the zcdk9 3'-UTR terminus is important for repression of *zcdk9* mRNA. (A) Terminal sequence of the *zcdk9* mRNA 3'-UTR and positions at which antisense MOs hybridize. The inventors defined the nucleotide at the junction of poly(A) tail as -1, and numbers were assigned accordingly. Total RNA was extracted from untreated (WT), cdk9 MO-injected and cdk9m MO-injected embryos. (B) Full repression of *zcdk9* mRNA by injection of cdk9 MO, MO-5, MO-6 and MO-7. Embryos in which indicated MOs were injected (lanes 2 to 8) or uninjected (lane 1) were collected at 3 hpf. Total RNA was extracted, and a PAT assay was performed with PAT primers for *cdk9* and *tbp.* (C) Extracts from embryos (5 hpf) in which indicated MOs were injected (lanes 2-8) or uninjected (lane 1) were analyzed by 7.5% SDS-polyacrylamide gel electrophoresis. Blots were probed with anti-zCdk9 antibody, anti-human Cdk9 antibody (H-169), and anti-actin antibody. HeLa cell nuclear extract (NE) was also analyzed (lane 9) as a control.
[Fig. 3]
   Determination of mRNA 3'-UTR terminal sequence. (A) Schematic drawings show the method employed in the present study to determine mRNA 3'-UTR terminal sequences. (B) PCR products were visualized by ethidium bromide staining. (C) Results of DNA sequencing analysis of each cDNA derived from MO-injected embryos. Five clones were selected, and the DNA sequences of their 3'-terminal sequences were aligned. The vertical line indicates the poly(A) tail junction of mRNA. The junction information was obtained from the NCBI nucleotide database (NM_212591.1.).
[Fig. 4]
   Specificity of poly(A) tail elongation inhibition by MO. (A) Sequences of cyclin B1 and B2 MOs. (B) Total RNA was extracted from 5 hpf embryos in which the indicated MOs were injected (lanes 2 to 4) or uninjected (lane 1). A PAT assay was performed with PAT primers for *cdk9, cyclin B1,* and *cyclin B2.* PCT assay using an *actin* primer set was also performed (actin). PCR products were analyzed on a 2.2% agarose gel. Right side: length markers in bases. (C) Total RNA was extracted from 5 hpf embryos in which the indicated MOs were injected (lanes 1 to 5) or uninjected (lane 6). A PAT assay was performed with PAT primers for *cdk9, tbp,* and *cyclin B1.* The PCR products were analyzed on a 2.2% agarose gel. Right side: length markers in bases.
[Fig. 5]
   Examination of *zcdk9* mRNA repression targeting *zcdk9* 3'-UTR. (A) Terminal sequence of the *zcdk9* mRNA 3'-UTR and positions at which antisense MOs hybridize. The inventors defmed the nucleotide to which the poly(A) tail is linked as -1, and numbers were assigned as shown in this Figure. (B) Only cdk9 MO affected the poly(A) tail elongation of *zcdk9* mRNA. Embryos into which indicated MOs were injected (lanes 1 to 5) or uninjected (lane 6) were collected at 3 hpf. Total RNA was isolated from each embryo. A PAT assay was performed with PAT primers for *cdk9* and *tbp,* and resultant PCR products were analyzed on a 2.2% agarose gel. Right side: length markers in bases. (C) Only cdk9 MO inhibited the translation of *zcdk9* mRNA. Embryos were collected at 5 hpf. Western blot analysis was performed with individual embryo extracts. HeLa cell nuclear extract (NE) was analyzed as a positive control (lane 7). Anti-zCdk9 antibody and anti-actin antibody were used.
[Fig. 6]
   Specific effect of MO on poly(A) tail shortening. (A) Sequence information for each of the mRNA 3'-UTRs indicated in this Figure and positions at which antisense MOs hybridize. (B) MO-mediated inhibition of poly(A) tail elongation detected in PAT assay. Embryos were collected at the times indicated. Total RNA was extracted from untreated (WT, lanes 21 to 24), cdk9 MO-injected (lanes 1 to 4), cdk9m MO-injected (lanes 5 to 8), tbp MO-injected (lanes 9 to 12), cyclin B1 MO-injected (lanes 13 to 16) and cyclin B2 MO-injected (lanes 17 to 20) embryos. PAT assay was performed with cdk9, tbp, cyclin B1, and cyclin B2 PAT primers; "oligo dT" indicates that oligo dT primers were used in reverse transcription; "tbp random" and "actin random" indicate that random primers were used in reverse transcription and that a tbp or actin primer set for PCR was used in the subsequent PCR reaction. The resultant PCR products were analyzed on a 2.2% agarose gel.
[Fig. 7]
   Hypothetical model in which the 3'-UTR terminal sequence of mRNA is determined after hybridization of MO. (A) Poly(A) tail shortening is activated by the hybridization of MO with the 3'-UTR of mRNA. After a deadenylase has removed the poly(A) tail, an exonuclease may gradually delete the 3'-UTR terminal sequence. In this model, the hybrid between MO and mRNA inhibits further invasion of the exonuclease. (B) An endonuclease recognizing the hybrid between MO and mRNA cleaves mRNA at a position downstream from the hybrid.
[Fig. 8]
   Cleavage of the poly(A) tail of target mRNA is induced in HeLa cells by mRNA 3'-UTR-targeting siRNA. Reverse transcription with oligo-dT primers and subsequent PCR with respective primers for RelA, Bcl-xL, Livin, PLK1, and actin. PCR products were analyzed on a 2.0% agarose gel. Total RNA was extracted from cells 24 hrs after siRNA transfer. Indicated by "n/c" are samples into which negative control siRNA was transferred.
[Fig. 9]
   Digestion of target mRNA is induced in HeLa cells by mRNA 3'-UTR-targeting siRNA. Reverse transcription with random primers and subsequent PCR with respective primers for Re1A, Bcl-xL, Livin, PLK1, and actin. PCR products were analyzed on a 2.0% agarose gel. Total RNA was extracted from cells 24 hrs after siRNA transfer. Indicated by "n/c" are samples into which negative control siRNA was transferred.
[Fig. 10]
   Translation inhibition of target gene is induced specifically in HeLa cells by mRNA 3'-UTR-targeting siRNA. Extracts from cells 24 hrs after each siRNA transfer were analyzed by western blotting with the antibodies indicated in this Figure. Indicated by "n/c" are samples into which negative control siRNA was transferred.
[Fig. 11]
   Cleavage of the poly(A) tail of *tPA* mRNA and digestion of the mRNA itself are induced in HeLa cells by *tPA* mRNA 3'-UTR-targeting siRNA. Reverse transcription with oligo-dT primers (upper row) and random primers (lower row) and subsequent PCR with respective primers for pTA and actin. PCR products were analyzed on a 2.0% agarose gel. Cells were treated with PMA of the indicated concentrations for 24 hrs. Total RNA was extracted from cells 24 hrs after siRNA transfer. Indicated by "n/c" are samples into which negative control siRNA was transferred.
[Fig. 12]
   Terminal sequence information for the mRNA 3'-UTR of each of RelA, Bcl-xL, Livin, PLK1, and tPA, and the target sequences (underlined portions) of siRNAs used in the experiment.

### BEST MODES FOR CARRYING OUT THE INVENTION

Hereinbelow, embodiments of the present invention will be described in more detail. The present invention provides a method of inhibiting the translation reaction of a target gene, comprising cutting out a part of the poly(A) tail and/or 3'-terminal sequence of the target mRNA.

The target gene of which the translation reaction is to be inhibited may be any gene. Examples of the target gene include, but are not limited to, enzyme genes, oncogenes, immunity-related genes, differentiation-related genes, nerve-related genes, DNA repair-related genes, and disease-related genes. Further, the target gene may be a gene whose function is not known.

For cutting out a part of the poly(A) tail and/or 3'-terminal sequence of the target mRNA, a ribonucleic acid may be used which is capable of hybridizing to the part of the poly(A) tail and/or 3'-terminal sequence of the target mRNA.

The ribonucleic acid capable of hybridizing to the part of the poly(A) tail and/or 3'-terminal sequence of the target mRNA may comprise a nucleotide sequence complementary to the whole or a part of a sequence encoding the region from the poly(A) tail junction of the target mRNA to the 45^{th} nucleotide upstream thereof. Preferably, the ribonucleic acid comprises a nucleotide sequence complementary to the whole or a part of a sequence encoding the region of 40 nucleotides from the poly(A) tail junction of the target mRNA to the 40^{th} nucleotide upstream thereof.

Further, the whole or a part of a sequence encoding the region of 40 nucleotides from the poly(A) tail junction of the target mRNA to the 40^{th} nucleotide upstream thereof may contain the whole of a part of a polyadenylation signal sequence.

The ribonucleic acid capable of hybridizing to a part of the poly(A) tail and/or 3'-terminal sequence of the target mRNA may be a 20- to 25-mer. The ribonucleic acid may be either a natural nucleotide (e.g., double-stranded RNA) or a nucleotide containing at least one nucleotide analogue. Examples of natural nucleotides include, but are not limited to, double-stranded RNA, DNA and DNA-RNA chimera. The ribonucleic acid containing at least one nucleotide analogue may be single-stranded. Examples of such ribonucleic acids include, but are not limited to, antisense morpholino oligonucleotide, S-oligo, 2'-O-methylated RNA, 2'-F-RNA, and BNA (LNA) oligo. Ribonucleic acids may be prepared by known methods such as genetic engineering techniques or chemical synthesis methods.

The method of the present invention for inhibiting translation reaction may be carried out *in vitro* (in cells or non-cellular systems) or *in vivo* (in organisms).

According to the method of the present invention for inhibiting translation reaction, it is possible to repress gene expression in cells, human, and non-human organisms. By using the method of the present invention for inhibiting translation reaction, it becomes possible, for example, to treat cancer by selective repression of cancer causative genes; to alleviate allergic response by specific repression of genes involved in immune reaction; to regulate differentiation by specific repression of genes involved in cell differentiation; and to develop psychotropic drugs by specific repression of genes involved in neuronal excitation transmission.

Further, the present invention provides a kit for inhibiting the translation reaction of a target gene, comprising a reagent capable of cutting out a part of the poly(A) tail and/or 3'-terminal sequence of the target mRNA. The reagent capable of cutting out a part of the poly(A) tail and/or 3'-terminal sequence of the target mRNA may be a ribonucleic acid. The ribonucleic acid may be one capable of hybridizing to a part of the poly(A) tail and/or 3'-terminal sequence of the target mRNA; and such a ribonucleic acid has been described above. The kit of the present invention may be used for repressing gene expression in cells, human, and non-human organisms. The kit of the present invention may further contain other reagents such as transfection reagents, control reagents (e.g., ribonucleic acid as negative control, and ribonucleic acid as positive control), reagents for detecting positive control (e.g., antibody to a protein that is targeted by positive control; and primers capable of detecting expression of the mRNA of the protein), manuals and the like.

Further, the present invention provides a cell in which the translation reaction of a target gene is inhibited by introduction thereinto of a reagent capable of cutting out a part of the poly(A) tail and/or 3'-terminal sequence of the target mRNA. In the cell of the present invention, expression of the target gene can be repressed.

The reagent capable of cutting out a part of the poly(A) tail and/or 3'-terminal sequence of the target mRNA may be a ribonucleic acid. The ribonucleic acid may be one capable of hybridizing to a part of the poly(A) tail and/or 3'-terminal sequence of the target mRNA; and such a ribonucleic acid has been described above.

For introduction into cells of a reagent that is capable of cutting out a part of the poly(A) tail and/or 3'-terminal sequence of the target mRNA, any gene transfer technique as exemplified by the calcium phosphate method, electroporation, lipofection, microinjection, the gene gun method, the Agrobacterium method or the virus vector method may be used,when the reagent is a ribonucleic acid. The ribonucleic acid capable of cutting out a part of the poly(A) tail and/or 3'-terminal sequence of the target mRNA may be introduced directly into cells or may be expressed in cells using a known vector system.

The cell into which a reagent capable of cutting out a part of the poly(A) tail and/or 3'-terminal sequence of the target mRNA is to be introduced may be any cell as long as it contains the target gene. Regardless of being differentiated or undifferentiated, the cell may be a somatic cell, germ cell, immortalized cell, or transformed cell. Specific examples of the cell include, but are not limited to, embryos (derived from human or non-human organisms), cancer cells, immune cells, nerve cells, germ cells, and stem cells. The cell may be derived from any organism, for example, animals such as fishes (zebrafish, etc.), mammals (human and non-human mammals such as mouse, rat, hamster, monkey, cattle, goat, pig, sheep, dog, etc.), birds (chicken, etc.), insects *(Drosophila,* etc.), echinoderms (urchin, starfish, sea cucumber, etc.), nematodes, frogs *(Xenopus laevis,* etc.); plants such as dicots *(Arabidopsis thaliana,* tobacco, cotton, etc.) and monocots (rice, corn, barley, wheat, etc.); bacteria such as *Escherichia coli* and *Bacillus subtilis;* and fungi such as molds and yeast.

Further, the present invention provides a non-human organism in which the translation reaction of a target gene is inhibited by introduction thereinto of a reagent capable of cutting out a part of the poly(A) tail and/or 3'-terminal sequence of the target mRNA. In the non-human organism of the present invention, expression of the target gene can be inhibited.

The reagent capable of cutting out a part of the poly(A) tail and/or 3'-terminal sequence of the target mRNA may be a ribonucleic acid. The ribonucleic acid may be one capable of hybridizing to a part of the poly(A) tail and/or 3'-terminal sequence of the target mRNA; and such a ribonucleic acid has been described above.

For introduction into organisms of a reagent that is capable of cutting out a part of the poly(A) tail and/or 3'-terminal sequence of the target mRNA, any gene transfer technique as exemplified by the calcium phosphate method, electroporation, lipofection, microinjection, the gene gun method, the Agrobacterium method or the virus vector method may be used when the reagent is a ribonucleic acid. The ribonucleic acid capable of cutting out a part of the poly(A) tail and/or 3'-terminal sequence of the target mRNA may be introduced directly into organisms or may be expressed in organisms using a known vector system.

The non-human organism into which a reagent capable of cutting out a part of the poly(A) tail and/or 3'-terminal sequence of the target mRNA is to be introduced may be any non-human organism as long as it contains the target gene. The non-human organism may be any organism selected from, for example, animals such as fishes (zebrafish, etc.), mammals (non-human mammals such as mouse, rat, hamster, monkey, cattle, goat, pig, sheep, dog, etc.), birds (chicken, etc.), insects *(Drosophila,* etc.), echinoderms (urchin, starfish, sea cucumber, etc.), nematodes, frogs *(Xenopus laevis,* etc.); plants such as dicots *(Arabidopsis thaliana,* tobacco, cotton, etc.) and monocots (rice, corn, barley, wheat, etc.); bacteria such as *Escherichia coli* and *Bacillus subtilis;* and fungi such as molds and yeast.

### EXAMPLES

Hereinbelow, the present invention will be described in more detail with reference to the following Examples. However, the present invention is not limited to these Examples.

### [Example 1]

### Evidence Showing that Antisense Morpholino Targeting Just Upstream from the Poly(A) Tail Junction of Maternal mRNA Removes the Tail and Inhibits Translation.

### Abstract

Gene downregulation by antisense morpholino oligonucleotides (MOs) is achieved by either hybridization around the translation initiation codon or by targeting the splice donor site. In this Example, an antisense MO method is introduced that uses a 25-mer MO against 40 nucleotides upstream from the poly(A) tail junction in the 3'-untranslated region (UTR) of a maternal mRNA. The inventors have found that the MO removed the poly(A) tail of *zcdk9* mRNA and inhibited its translation. This shows functional mimicry between miRNA and MO. A PCR-based assay detected that mRNAs of zebrafish *cdk9, tpb, cyclin B1,* and *cyclin B2* undergo specific MO-mediated poly(A) tail elongation inhibition. Therefore, the antisense method introduced in this Example revealed that MO has miRNA-like activity in the regulation of mRNA and, at the same time, showed that MO is applicable to downregulation of maternal mRNAs in animal oocytes and early embryos.

### Introduction

The antisense method of gene downregulation classically utilizes DNA - RNA duplex formation of single-stranded (ss) DNA through complementary base pairing, leading to RNase H-mediated cleavage of the target mRNA *in vivo.* However, endonucleases that efficiently digest ssDNAs exist *in vivo,* decreasing the antisense activities by ssDNAs. In order to avoid this problem, morpholino oligonucleotides (MOs) are frequently used because they are endonuclease resistant. While antisense ssDNAs induce gene downregulation by digestion of target mRNA, MOs do not mediate the RNase H-mediated digestion of mRNAs. Briefly, duplex formation between MOs and mRNA prevents translation through MO hybridization near the mRNA translation initiation codon or prevents correct splicing by duplex formation at the splice donor site. In both cases, antisense MOs are used as a very useful means for gene downregulation, but it is difficult to confirm MO-mediated specific inhibitory effect against gene expression.

In this Example, a novel method for gene downregulation is described. The efficacy and specificity of this method were confirmed by targeting maternal mRNAs of zebrafish *cdk9, tbp, cyclin B1,* and *cyclin B2.* The key features of this method are as follows: 1) duplex formation between MO and the mRNA 3'-untranslated region (UTR) not only inhibits poly(A) tail elongation, but also deletes the poly(A) tail and 2) this method blocks target mRNA translation. An important point is that MOs targeting at 3'-UTR behave like miRNAs that induce poly(A) tail shortening and translation inhibition simultaneously.

### Materials and Methods

### • Synthesis

DNAs and morpholino oligonucleotides were synthesized by Operon Biotechnologies and Gene Tools, respectively.

### • Embryos

All zebrafish and embryos were bred at 28°C.

### • Microinjection of MO

Zebrafish wild-type embryos were injected at one- or two-cell embryo stage with approx. 2.5 pmol of MO. The MOs used in this study are shown below.
Antisense Morpholino Oligonucleotides
cdk9 MO: GGAAATGTGAAGGATTTATAGGTGT (SEQ ID NO: 1)
cdk9 MO-2: ATTTATACTTATACAAGTAACAAAC (SEQ ID NO: 2)
cdk9 MO-3: ACCATGACCCCGAACACGTGATCTT (SEQ ID NO: 3)
cdk9 MO-4: ACAAATAAAAACATCTTTAAAAATA (SEQ ID NO: 4)
cdk9 MO-5: TGTGAAGGATTTATTGGTGTATTTA (SEQ ID NO: 5)
cdk9 MO-6: AGGATTTATTGGTGTATTTATACTT (SEQ ID NO: 6)
cdk9 MO-7: TTATTGGTGTATTTATACTTATACA (SEQ ID NO: 7)
cdk9 MO-8: GGTGTATTTATACTTATACAAGTAA (SEQ ID NO: 8)
cdk9m MO: GGTAATATGAACGATGTATAGGTGT (SEQ ID NO: 9)

When a mixture of two MOs was to be examined, 1.25 pmol of each MO was injected into embryos.

### • Preparation of Extracts from Embryos

Ten zebrafish embryos collected were frozen in liquid nitrogen and thawed in 200 µl of RIPA buffer [150 mM NaCl, 1% NP-40, 0.5% deoxycholate, 0.1% sodium dodecyl sulfate, 50 mM Tris-HCl (pH 8.0)]. After thorough sonication, supernatants (100 µl) were collected by centrifugation at 12,000g for 1 min and mixed with 40 µl of 4x Laemmli sample buffer. In western blot analysis, 14 µl of each sample (corresponding to a half-embryo) was analyzed.

### • Purification of Embryo-Derived Total TNA

Total RNA was prepared from embryos with Sepasol-RNA I super (Nacalai Tesque).

### • Poly(A) Test Assay (PAT)

The PAT assays were performed essentially as described in Reference Document No. 10, except for minor modifications. Total RNA (300 ng) was incubated at 65°C for 5 min in the presence of a mixture of phosphorylated oligo (dT) primers, which were 12- to 18-mer poly (dT) primers. After incubation for 1 h at 42°C with T4 DNA ligase (350 U) (TaKaRa Bio), the samples were further incubated at 12°C for 1 h in the presence of 200 ng of (dT)₁₂-anchor primer (5'-GCGAGCTCCGCGGCCGCGTTTTTTTTTTTT-3' (SEQ ID NO: 10)) and then incubated at 42°C for 1 h with SuperScript III reverse transcriptase (200 U) (GE Healthcare) to thereby obtain PAT cDNAs. Finally, PCR was performed using (dT)₁₂-anchor primer and respective gene-specific primers. PCR products were subjected to 2.2% agarose gel electrophoresis, followed by visualization of DNA bands with ethidium bromide. The gene-specific primers used in this study are shown below.

### Primers for PCR Assay

| | |
|---|---|
| zcdk9 PAT | GTGCTGCCCCAGTGCATTGT (SEQ ID NO: 11) |
| tbp PAT | TGTTGTGCAGTGCGAGAGATC (SEQ ID NO: 12) |
| cyclin B1 PAT | ATGTTGTGAGGGTCAACGAGG (SEQ ID NO: 13) |
| cyclin B2 PAT | AGCAGCAGACTCATGAAGATCA (SEQ ID NO: 14) |

### • Reverse Transcription-PCR (RT-PCR)

RT-PCR was performed with SuperScript III transcriptase (200 U). Random and oligo (dT) primers (Invitrogen) were used as reverse primers. The gene-specific primer sets (forward and reverse primers) used in RT-PCR are shown below.

### PCR Primers

actin forward: 5'-CTGAATCCCAAAGCCAACAG-3' (SEQ ID NO: 15)
actin reverse: 5'-TCACACCATCACCAGAGTCC-3' (SEQ ID NO: 16)
biklf forward: 5'-ATGCTGACTCCACCATCCTC-3' (SEQ ID NO: 17)
biklf reverse: 5'-TGTCCGGTGTGTTTCCTGTA-3' (SEQ ID NO: 18)
zcdk9 forward: 5'-CAGCCAATCAGAGTTCGACA-3' (SEQ ID NO: 19)
zcdk9 reverse: 5'-TAGTGCCACCGGTAAACTCC-3' (SEQ ID NO: 20)
tbp forward: 5'-CTTGGGGTGCAAACTTGATT-3' (SEQ ID NO: 21)
tbp reverse: 5'-CATATTTCCTGGCTGCCAAT-3' (SEQ ID NO: 22)
cyclin B1 forward: 5'-CAGCTGCAACTTGTTGGTGT-3' (SEQ ID NO: 23)
cyclin B1 reverse: 5'-GGTAGAGGCCTTCCAAAACC-3' (SEQ ID NO: 24)
cyclin B2 forward: 5'-CTCAAAGCATCTGACGGTGA-3' (SEQ ID NO: 25)
cyclin B2 reverse: 5'-GCAGCAGTCCATCTCTCACA-3' (SEQ ID NO: 26)

### • Western Blot Analysis

To generate anti-zCdk9 antibodies, recombinant zCdk9 protein was expressed in *Escherichia coli* and fractionated by disk preparative electrophoresis. Rabbits (entrusted to TaKaRa Bio) were immunized with the Cdk9 protein and treated according to the standard protocol of Operon Biotechnologies. Polyclonal anti-zCdk9 antibodies were purified as described in Reference Document No. 11. Anti-human Cdk9 antibody (H-169) and anti-actin antibody (clone C4) were purchased from Santa Cruz Biotechnology and Chemicon, respectively. Immunoblotting was performed as described in Reference Document No. 12. Blots were developed with the ECL system (GE Healthcare).

### • Determination of the 3'-Terminal Sequence of mRNA

Small RNA Cloning Kit (Takara) was used according to the manufacturer's manual with minor modifications. Three hundred nanograms of total RNA isolated from embryos was treated with alkaline phosphatase (BAP). A biotinylated RNA/DNA 3' adaptor was ligated to 3'-terminally BAP-treated RNA. Streptavidin-conjugated magnetic beads were used to collect the adaptor-ligated RNA. After washing the beads, reverse transcription was performed with PCR-R & RT-primer. Synthesized cDNAs were recovered from the beads by alkaline treatment, followed by PCR with *zcdk9* PAT primer and PCR-R & RT-primer. The PCR products were subjected to agarose gel electrophoresis, and DNA bands were visualized by ethidium bromide staining. The bands were cut out. The DNA fragments were eluted and then cloned into pMD20-T vector. After blue-white selection of clones on agar plates containing ampicillin, plasmid DNA was recovered from each clone. Insert regions were confirmed by restriction enzyme digestion. More than 10 clones were selected from each sample for further DNA sequencing analysis.

### Results

### • Injection of MO targeting the 3'-UTR terminus of zcdk9 mRNA into zebrafish early embryos inhibits poly(A) tail elengation and subsequent translation reaction.

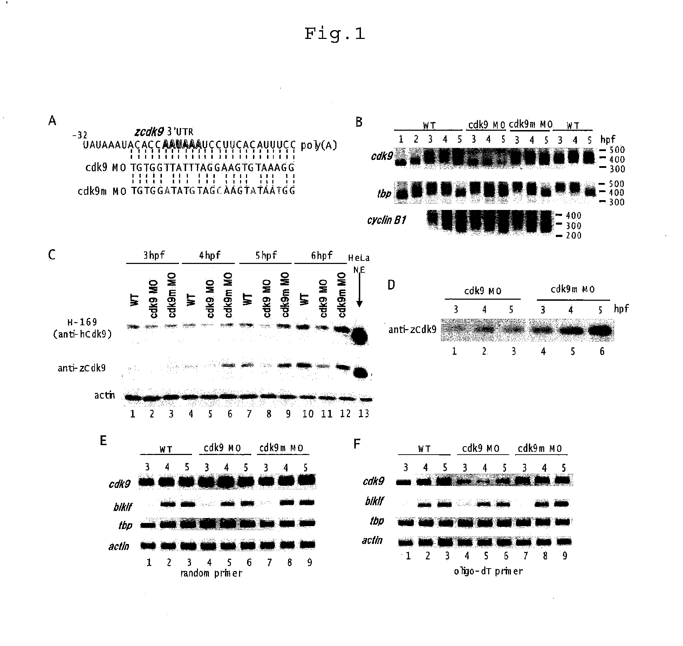

In the course of analysis of the expression regulation mechanism of zebrafish kinase *cdk9* mRNA at the midblastula transition, the inventors noticed that elongation of the poly(A) tail plays a critical role in translation stimulation. To address this finding, the inventors invented an antisense experiment with a low-toxicity nucleoside analogue, morpholino oligonucleotide (MO). The inventors predicted that duplex formation between an MO and the 3'-UTR of mRNA should inhibit poly(A) tail elongation. The inventors prepared cdk9 MO consisting of 25 nucleotides that were completely complementary to the terminal sequence of the *zcdk9* mRNA 3'-UTR (Fig. 1A). To examine whether MOs against the 3'-UTR affect poly(A) tail elongation, a PAT assay was performed in which the PCR products reflect the poly(A) tail lengths of specific mRNAs. When injected into fertilized embryos, cdk9 MO markedly reduced slowly migrating bands compared to such bands in untreated embryos (WT), suggesting that cdk9 MO inhibited poly(A) tail elongation (Fig. 1B). The cdk9 MO also strongly reduced the amount of *cdk9* band, without affecting the products of *tbp* and *cyclin B1.* It should be noted here that this result was specific for cdk9 MO, because embryos treated with cdk9m MO carrying 5-base mismatches in the hybridizing nucleotide sequence produced no different result from the result of untreated embryos (Fig. 1B). These results demonstrate that cdk9 MO specifically affects *cdk9* mRNA.

Subsequently, the inventors assessed the gene downregulation effect of MOs at the protein level. A western blot assay was performed with two independent antibodies (anti-zCdk9 antibody against zebrafish Cdk9 originally created by the inventors and the commercially available H-169 antibody against human Cdk9). Anti-actin antibody was used as a control. The cdk9 MO did not affect zCdk9 accumulation in 3 hpf embryos, but blocked the zCdk9 increment that began at 4 hpf (Fig. 1C). Unlike cdk9 MO, cdk9m MO showed no detectable significant difference in the translation reaction of *zcdk9* mRNA. In order to show these results more clearly, western blot assay was performed with the samples used in Fig. 1C. As a result, it was possible to show that cdk9 MO inhibits increase of Cdk9 protein accumulation in early embryos (Fig. 1D). Taken together, these results indicate that injection of cdk9 MO inhibits the translation of maternal *zcdk9* mRNA at an early stage of development.

Since the levels of *cdk9* products in the PAT assay were decreased by the injection of cdk9 MO, the inventors investigated the effect of cdk9 MO by RT-PCR. Reverse transcription was performed with either random primers or oligo-dT primers, followed by PCR amplification of the *zcdk9-*coding region. In order to confirm the experimental time course, the inventors analyzed the expression of the gene *bilkf* which became detectable at 3 hpf and began showing mRNA accumulation at 4 hpf (Fig. 1E and F). The results show that transcription from somatic cell genes occurs at around 3 hpf, corresponding approximately to the beginning of cleavage cycle 10. This result was consistent with previously reported experimental facts. RT-PCR with random primers yielded the same amount of *zcdk9* products in the mRNAs extracted from all embryos used in the experiment, regardless of cdk9 MO injection (Fig. 1E), which indicates that injection of cdk9 MO into embryos does not decrease the amount of *zcdk9* mRNA. On the other hand, in the experiment using oligo-dT primers, RT-PCR analysis of cdk9 MO-injected embryos revealed a decrease in *zcdk9* mRNA aggregation (Fig. 1F). Since oligo-dT primers hybridize to the poly(A) tail, hybridization efficiency decreases in short poly(A) tails consisting of a few adenosines, presumably leading to a lower amplification efficiency. Therefore, the low-level amplification of DNA fragments observed in RT-PCR assay may have been due to production of short poly(A) tails caused by poly(A) tail shortening (see below). These results indicate that injection of cdk9 MO into early embryos induces shortening of the poly(A) tail of mRNA rather than mRNA degradation. • The region of 40 nucleotides from the *zcdk9* 3'-UTR terminus is an active region in MO-mediated inhibition ofpoly(A) tail elongation.

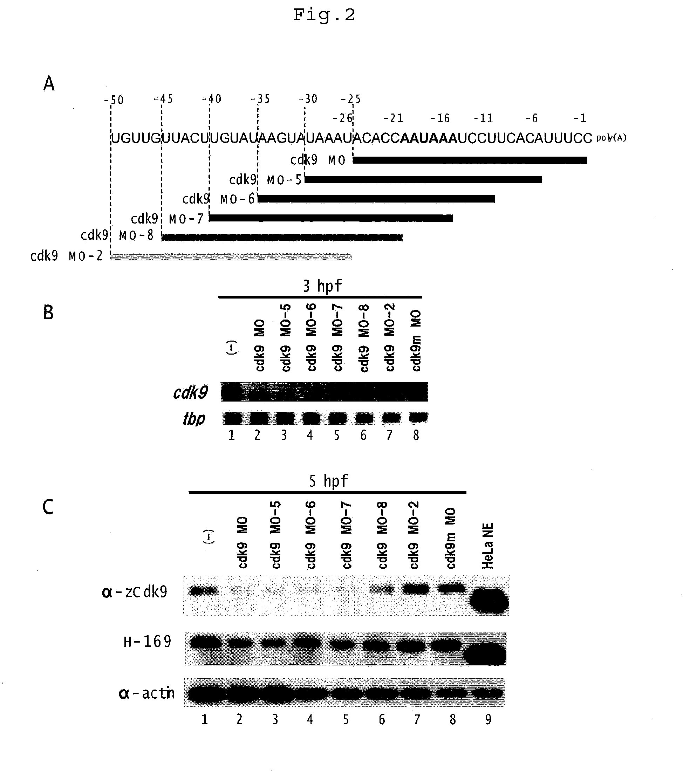

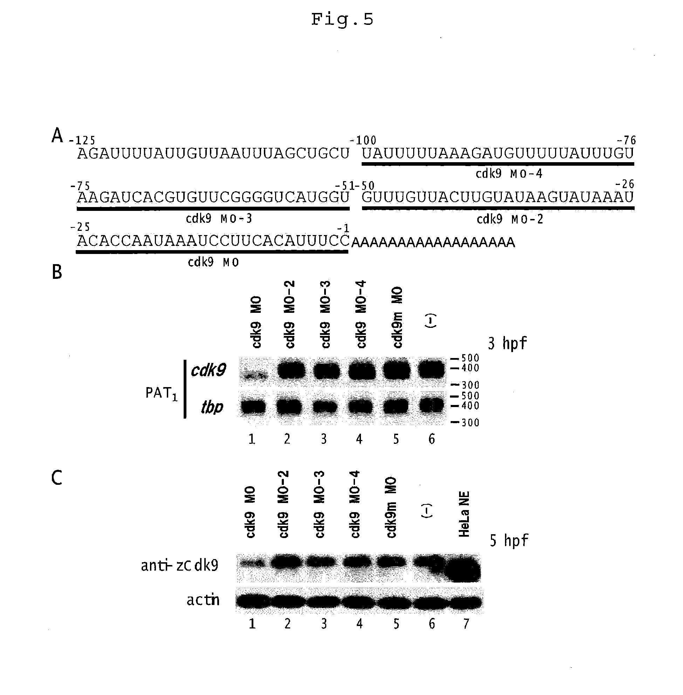

To address whether the 3'-UTR terminus plays an important role in MO-mediated repression, three antisense MOs were created: MO-2 (-26 to -50), MO-3 (-51 to -75), and MO-4 (-76 to -100) (Fig. 5). The PAT assay and western blot analysis revealed that, of the five MOs, only cdk9 MO exerted an inhibitory effect on both poly(A) tail elongation and translation. This finding suggests that the most terminal portion of the 3'-UTR is critical for the inhibition. To verify this result, four additional MOs were created and examined. The results confirmed that MO-5 (-6 to -30), MO-6 (-11 to -35) and MO-7 (-16 to 40) show inhibitory effects on both poly(A) tail elongation and translation. A modest inhibitory effect on both poly(A) tail elongation and translation was observed when MO-8 (-21 to -45) was examined (Fig. 2B and C). These results indicate that the terminal 40 nt of 3'-UTR act as the active site in MO-mediated inhibition.

### • Evidence showing that antisense MO completely removes the poly(A) tail from zcdk9 mRNA

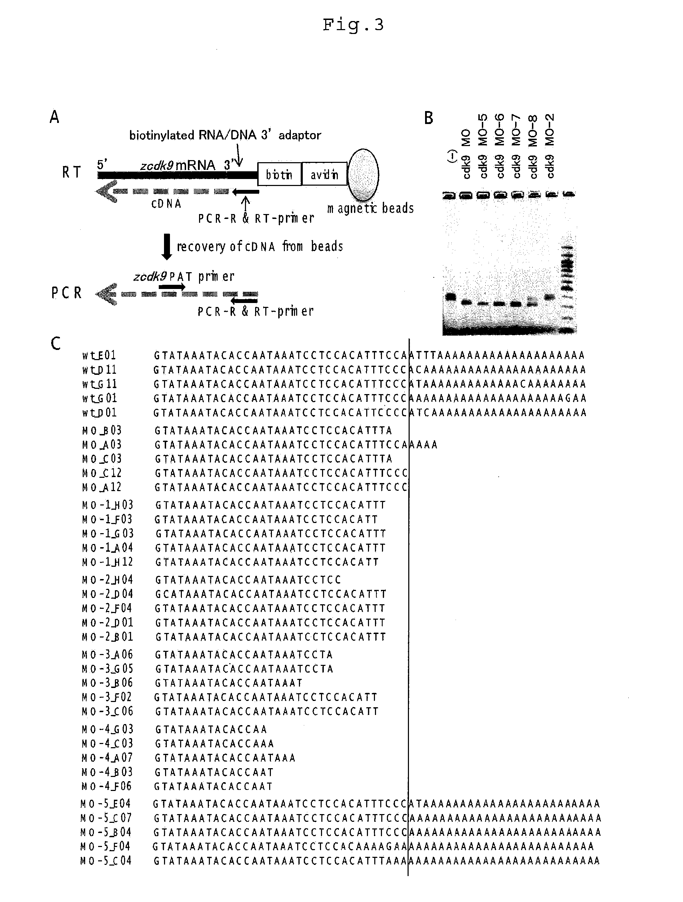

As described earlier, duplex formation between MO and the mRNA 3'-UTR terminus inhibits proper poly(A) tail elongation. To determine precisely how many adenosine residues remain in the poly(A) tail after injection of MO, the inventors performed DNA sequencing of the 3'-terminal region of *zcdk9* cDNAs synthesized from mRNAs derived from MO-injected embryos obtained in Fig. 2. For this sequencing, small RNA Cloning Kit (Takara) was used. In this kit, a biotinylated RNA/DNA 3' adaptor was ligated to the 3'-terminus of mRNA. Then, the adaptor-ligated mRNA is collected and purified with streptavidin-conjugated magnetic beads. Reverse transcription is performed on those beads. After recovery of synthesized cDNAs, PCR was performed with *zcdk9* PAT primer and PCR-R & RT-primer to amplify the cDNAs (Fig. 3A). The PCR products were analyzed by agarose gel electrophoresis, and DNA bands were visualized by ethidium bromide staining (Fig. 3B).

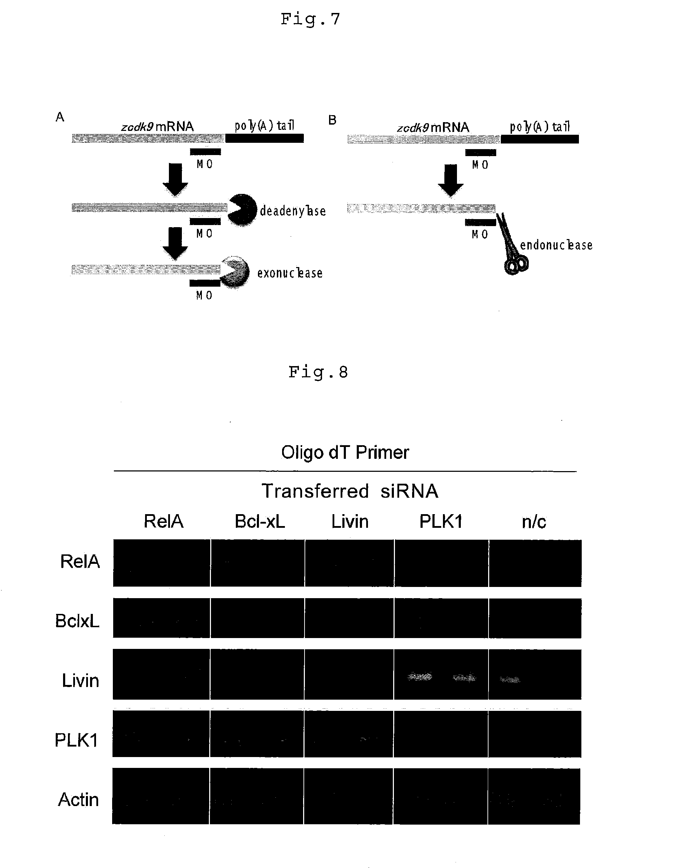

These PCR products were then cloned into the TA-cloning vector pMD20-T (TaKaRa) and DNA sequencing analysis of the cloned regions was performed. As shown in Fig. 3C, zcdk9 cDNA derived from MO-2-injected embryos had an intact poly(A) tail, similar to that seen in untreated embryos. In contrast, cDNAs from embryos that had been injected with MO, MO-5, MO-6, MO-7 or MO-8 lost the entire poly(A) tail, indicating that injection of MO into early embryos leads to removal of the poly(A) tail from the mRNA targeted by MO. Further, the inventors have noticed that the 3'-terminal sequences of the cDNAs were not identical, and also noticed that the remaining 3'-terminal sequence moves upstream as the MO-mRNA hybridization position moves upstream. Briefly, there is a tendency that the 3'-terminus moves more upstream in the following order: MO<MO-5<MO-6<MO-7<MO-8. This finding suggests that the hybridization position between MO and mRNA contributes to the determination of the 3'-terminal sequence of each mRNA. This effect may be due to the joint activity of deadenylase and exonuclease or to the action of an endonuclease recognizing the hybrid (see Discussion and Fig. 7).

### • Analysis of the effects of MO on four maternal mRNAs

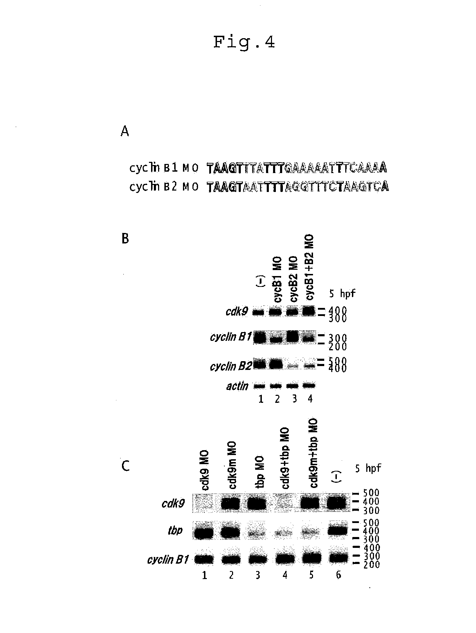

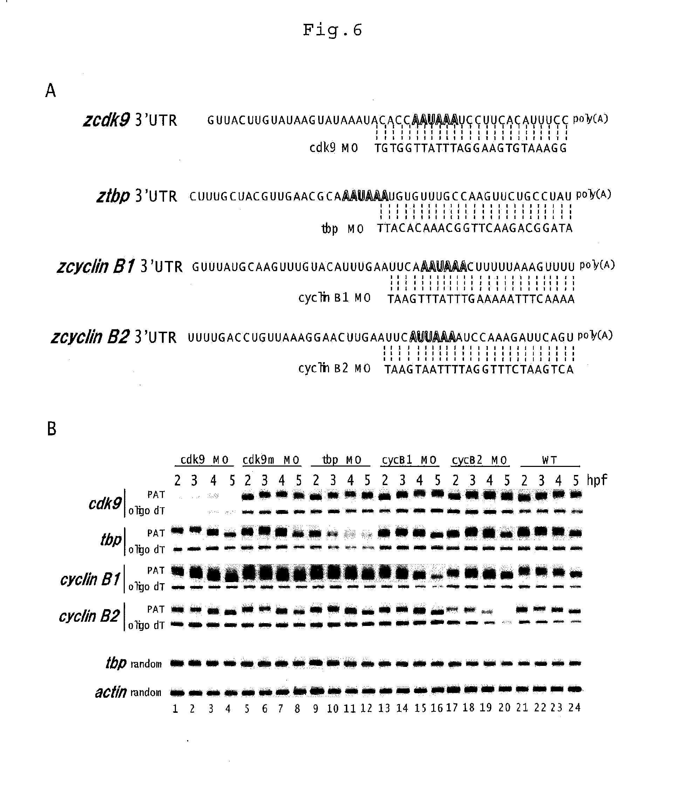

To evaluate the effect of MO on poly(A) tail elongation, three gene-specific MOs (cyclin B1 MO, cyclin B2 MO, and tbp MO) were created in addition to cdk9 MO (Fig. 4). To determine the MO specificity simply, the total RNA was extracted from 5 hpf embryos, and a PAT assay was performed (Fig. 4B and C). Cyclin B1 MO and cyclin B2 MO were also examined (Fig. 4A and B). It should be noted here that Zebrafish has the *cyclin B* orthologs *B1* and *B2,* and their 3'-UTRs exhibit sequence similarity (Fig. 4A) Indeed, there were 10 homologous nucleotides between cyclin B1 MO and cyclin B2 MO (Fig. 4A). The experimental results showed that cyclin B1 MO and cyclin B2 MO each affected its own target mRNA in a specific manner (Fig. 4B). The inventors also observed the specific action of MO when cdk9 MO and tbp MO were examined (Fig. 4C). The effects of MOs on the poly(A) tail elongation of mRNAs were further confirmed by using mRNA samples collected from 2 to 5 hpf embryos in Fig. 6A and B. These results indicate that the antisense MO method targeting the 3'-UTR of mRNAs affect poly(A) tail elongation in early zebrafish embryos in a specific manner.

### Discussion

The inventors report the inhibition of expression of maternal mRNAs in zebrafish early embryos. This inhibition is caused by inhibition of poly(A) tail elongation and inhibition of translation. With respect to inhibition of poly(A) tail elongation, it is believed that duplex structure between MO and mRNA as formed upstream from the junction of the poly(A) tail of the mRNA is necessary for MO-mediated inhibition ofpoly(A) tail elongation (Figs. 2 and 5). Notably, when MO hybridizes with a region spanning from 26 nt to 50 nt upstream from the 3'-UTR terminus of *zcdk9* mRNA, no effect is observed on the poly(A) tail (Figs. 2 and 3). This result suggests that the terminal 25 nucleotides of 3'-UTR region are an important *cis*-element. The 3'-terminal 25 nucleotides of *zcdk9, cyclins B1* and *B2* mRNAs and the 3'-terminal 30 nucleotides of *tbp* mRNA have a typical polyadenylation signal (AAUAAA), to which CPSF (one of the RNA-protein complexes involved in cytoplasmic polyadenylation) binds. In *Xenopus* oocytes, the RNA-protein complex comprises cytoplasmic poly(A) ribonuclease (PARN) and the cytoplasmic poly(A) polymerase (Gld-2), in addition to CPSF and cytoplasmic polyadenylation factors, including CPEB, Pumilio, and Musashi. Therefore, it is plausible that hybridization of MO to the poly(A) tail junction may prevent the proper binding of CPSF to the polyadenylation signal and the function of the RNA-protein complex. This process may possibly lead to perturbation of the balance between Gld-2 and PARN, resulting in poly(A) tail shortening.

The inventors have found that hybridization between MO and mRNA leads to removal of a 3'-terminal sequence and the entire poly(A) tail from the mRNA. Briefly, poly(A) tails is removed since mRNA is cleaved at a position several nucleotides downstream from the hybrid position (Fig. 3). It is believed that this removal is caused by deadenylase-mediated shortening of the poly(A) tail, followed by exonuclease action (Fig. 7A). Alternatively, hybridization may stimulate the activity of a certain endonuclease, leading to cleavage of the mRNA at the region downstream from the hybrid position (Fig. 7B). The inventors do not have a definite answer as to which hypothesis fits the present case. If the latter hypothesis fits, it is necessary to propose a new model showing involvement of an endonuclease which recognizes a MO-mRNA hybrid and binds thereto.

There are three important similarities between miRNA and MO behaviors. Briefly, the 3'-UTR of mRNA is the target; poly(A) tail shortening occurs; and translation inhibition occurs. Therefore, it will be of considerable interest to determine whether or not miRNA-related factors are involved in MO-mediated poly(A) tail shortening.

A novel method using MO has been introduced in this Example. The advantages of this method are as follows. Since MO is targeting the 3'-UTR of mRNA, designing is simpler than in conventional methods; and it is possible to confirm the effect of MO efficiently. Since the inventors possessed a specific antibody to zebrafish Cdk9, it was possible to confirm the quantitative decrease of both proteins induced by MO. When an appropriate antibody is not available, it is possible to examine inhibition of gene expression through monitoring the length of poly(A) tail by a PCR-based PAT assay. In addition to the validation at the RNA level, it is also possible to confirm the effect of MO on expression of the target gene by carrying out a recovery experiment with synthetic mRNAs as performed in Fig. 4F.

### References

1. Minshull, J. and Hunt, T. (1986) The use of single-stranded DNA and RNase H to promote quantitative 'hybrid arrest of translation' of mRNA/DNA hybrids in reticulocyte lysate cell free translations. Nucleic Acids Res., 14, 6433-651.
2. Cerritelli, S.M. and Crouch, R.J. (2009) Ribonuclease H: the enzymes in eukaryotes. FEBS J., 276, 1494-1505.
3. Summerton, J. (1999) Morpholino antisense oligomers: the case for an RNase H-independent structural type. Biochim. Biophys. Acta., 1489, 141-158 .
4. Eisen, J.S. and Smith, J.C. (2008) Controlling morpholino experiments: don't stop making antisense. Development, 135, 1735-1743.
5. Bill, B.R., Petzold, A.M., Clark, K.J., Schimmenti, L.A. and Ekker, S.C. (2009) A primer for morpholino use in zebrafish. ZEBRAFISH, 6, 69-77.
6. Richter, J.D. (2007) CPEB: a life in translation. TRENDS Biochem. Sciences, 32, 279-285.
7. Radford, H.E., Meijer, H.A. and de Moor, C.H. (2008) Translational control by cytoplasmic polyadenylation in Xenopus oocytes. Biochim. Biophys. Acta., 1779, 217-229.
8. Filipowicz, W, Bhattacharyya, S.N. and Sonenberg, N. (2008) Mechanisms of post-transcriptional regulation by microRNAs: are the answers in sight? Nat. Rev. Genet., 9, 102-114.
9. Westerfield, M. (1995) The Zebrafish Book: A guide to the Laboratory Use of Zebrafish (Danio rerio), 3 Edition.: Eugene: University of Oregon Press.
10. Salles, F.J. and Strickland, S. (1995) Rapid and sensitive analysis of mRNA polyadenylation states by PCR. PCR Methods Appl., 4, 317-321.
11. Harlow, E. and Lane, D. (1988) Antibodies: A laboratory manual. Cold Spring Harbor Laboratory, Cold Spring Harbor, NY
12. Wada, T., Takagi, T., Yamaguchi, Y, Ferdous, A., Imai, T., Hirose, S., Sugimoto, S., Yano, K., Hartzog, G.A., Winston, F., Buratowski, S. and Handa, H. (1998) DSIF, a novel transcription elongation factor that regulates RNA polymerase II processivity, is composed of human Spt4 and Spt5 homologs. Genes Dev., 12, 343-356.
13. Okano-Uchida, T., Sekiai, T., Lee, K., Okumura, E., Tachibana, K. and Kishimoto, T. (1998) In vivo regulation of cyclin A/Cdc2 and cyclin B/Cdc2 through meiotic and early cleavage cycles in starfish. Dev. Biol., 197, 39-53.
14. Okano-Uchida, T., Okumura, E., Iwashita, M., Yoshida, H., Tachibana, K. and Kishimoto, T. (2003) Distinct regulators for Plkl activation in starfish meiotic and early embryonic cycles. EMBO J., 22, 5633-5642.
15. Kishimoto T. (1986) Microinjection and cytoplasmic transfer in starfish oocytes. Methods Cell Biol., 27, 379-394.
16. Tachibana, K., Ishiura, M., Uchida, T., and Kishimoto, T. (1990) The starfish egg mRNA responsible for meiosis reinitiation encodes cyclin. Dev. Biol., 140, 241-252
17. Kane D.A. and Kimmel C.B. (1993) The zebrafish midblastula transition. Development, 119, 447-456.
18. O' Boyle, S., Bree, R.T., McLoughlin, S., Grealy, M. and Byrnes, L. (2007) Identification of zygotic genes expressed at the midblastula transition in zebrafish. Biochem. Biophys. Res. Comm., 358, 462-468.
19. Mathavan, S., Lee, S.G.P., Mak, A., Miller, L.D., Murthy, K.R.K., Govindarajan, K.R., Tong, Y, Wu, YL., Lam, S.H., Yang, H., Ruan, Y, Korzh, V, Gong, Z., Liu, E.T. and Lufkin, T. (2005) Transcriptome analysis of zebrafish embryogenesis using microarrays. PLoS Genet., 1, 260-276.
20. Hara, M., Mori, M., Wada, T., Tachibana, K. and Kishimoto, T. (2009) Start of the embryonic cell cycle is dually locked in unfertilized starfish eggs. Development, 136, 1687-1696.
21. Tachibana, K., Machida, T., Nomura, Y and Kishimoto, T. (1997) MAP kinase links the fertilization signal transduction pathway to the G1/S-phase transition in starfish eggs. EMBO J., 16, 4333-4339.
22. Kim, J.H. and Richter, J.D. (2006) Opposing polymerase-deadenylase activities regulate cytoplasmic polyadenylation. Mol. Cell, 24, 173-183.

### [Example 2]

### Materials and Methods

### • Cell Culture

HeLa cells were cultured in DMEM (Sigma-Aldrich) containing 10% inactivated FBS, 100 units/mL penicillin, and 100 mg/mL streptomycin at 37°C under 5% CO₂.

### • Gene Transfer

Cells were suspended in 10% inactivated FBS-containing DMEM to give a concentration of approx. 1 x 10⁶ cells/mL. The resultant suspension was plated on 12-well plates at 1 mL/well. When cells reached about 40-50% confluence, a mixture of siRNA (final concentration: 20 nM), 2 µL of Lipofectamine (Invitrogen), and 200 µL of OptiMEM (Invitrogen) was added to the culture. When necessary, PMA (final concentration: 10 nM or 100 nM) was also added. Cells were harvested 24 hrs after the addition. Total RNA was purified or subjected to western blot analysis.

The siRNAs used in this study are shown below.

### siRNAs

Re1A: 5'-CUGAACUAAUAAAUCUGUU -3'(SEQ ID NO: 27)
Bcl-xL: 5'-GUUCAGUAAUAAACUGUGU -3' (SEQ ID NO: 28)
Livin: 5'-GAAUAGAAAUAAAGUGGGU -3' (SEQ ID NO: 29)
PLK1: 5'-UAUGCACAUUAAACAGAUG -3' (SEQ ID NO: 30)
tPA: 5'-CUGUACUUAAUAAAUUCAG -3' (SEQ ID NO: 31)
n/c: Universal negative control (Nippon EGT)

### • Purification of Total RNA from Cells

Total RNA was prepared from cells using Sepasol-RNA I super (Nacalai Tesque).

### • Reverse Transcription-PCR (RT-PCR)

RT-PCR was performed using SuperScript III transcriptase (200 U). Random and oligo (dT) primers (Invitrogen) were used as reverse transcription primers. Gene-specific primers for RT-PCR (forward and reverse primers) are shown below.

### PCR Primers

RelA forward: 5'-CCTGGAGCAGGCTATCAGTC -3' (SEQ ID NO: 32)
RelA reverse: 5'-ATCTTGAGCTCGGCAGTGTT-3'(SEQIDNO: 33)
Bcl-xL forward: 5'-GGTATTGGTGAGTCGGATCG -3' (SEQ ID NO: 34)
Bcl-xL reverse: 5'-AAGAGTGAGCCCAGCAGAAC -3' (SEQ ID NO: 35)
PLK1 forward: 5'-GGCAACCTTTTCCTGAATGA-3' (SEQ ID NO: 36)
PLK1 reverse: 5'-AATGGACCACACATCCACCT -3' (SEQ ID NO: 37)
tPA forward: 5'-CCCAGATCGAGACTCAAAGC -3' (SEQ ID NO: 38)
tPA reverse: 5'-TGGGGTTCTGTGCTGTGTAA -3' (SEQ ID NO: 39)
Livin forward: 5'-CCTCTCTGCCTGTTCTGGAC -3' (SEQ ID NO: 40)
Livin reverse: 5'-CTCCAGGGAAAACCCACTTT -3' (SEQ ID NO: 41)
β Actin forward: 5'-GATATCGCCGCGCTCGTCG -3' (SEQ ID NO: 42)
β Actin reverse: 5'-GGGAGGAGCTGGAAGCAG -3' (SEQ ID NO: 43)

### • Western Blot Analysis

Harvested cells were washed with 1 mL of PBS and then dissolved in 125 µL of RIPA buffer (150 mM NaCl, 1% NP-40, 0.5% deoxycholate, 0.1% sodium dodecyl sulfate, 50 mM Tris-HCl [pH 8.0]). Supernatant (62.5 µL) was recovered by centrifugation (15,000 rpm) for 5 min, followed by addition of 62.5 µL of 2x Laemmli sample buffer thereto. Western blot analysis was performed on 5 µL samples. As primary antibody, anti-Bcl-xL antibody (Santacruz, sc-8392), anti-PLK1 antibody (Santacruz, sc-17783), anti-Livin antibody (Santacruz, sc-30161), anti-RelA antibody (Santacruz, sc-372) or anti-actin antibody (Millipore, clone C4) was used. As secondary antibody, HRP-conjugated anti-rabbit IgG (GE Healthcare, NA9340OV) or POD-conjugated mouse IgG to Rabbit IgG (Dako, P0260) was used. Responsive proteins were treated with SuperSignal West Pico Chemiluminescent Substrate (Pierce) and visualized with LAS4000 IR multi color (Fuji Film).

### Results

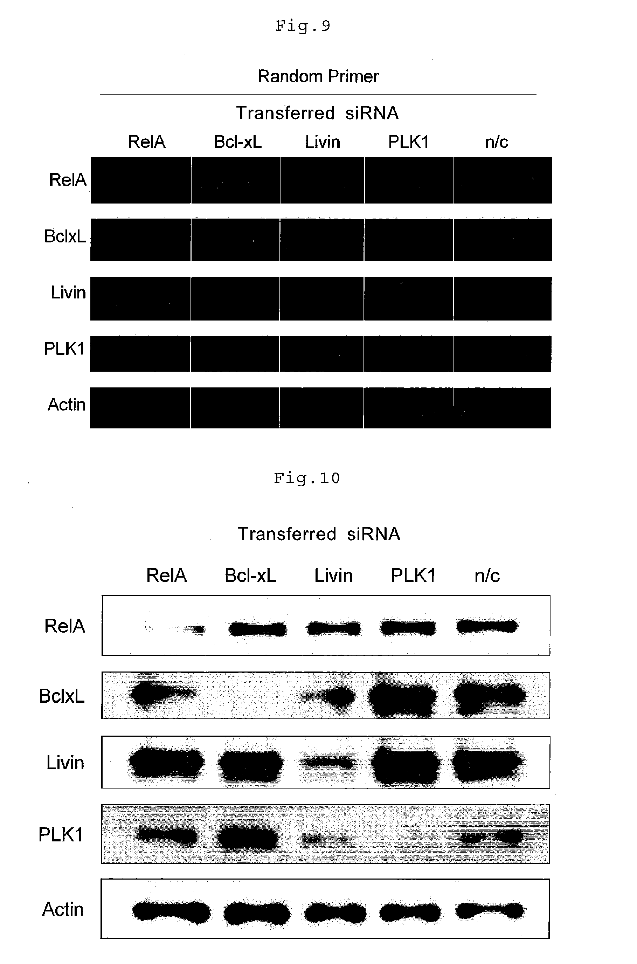

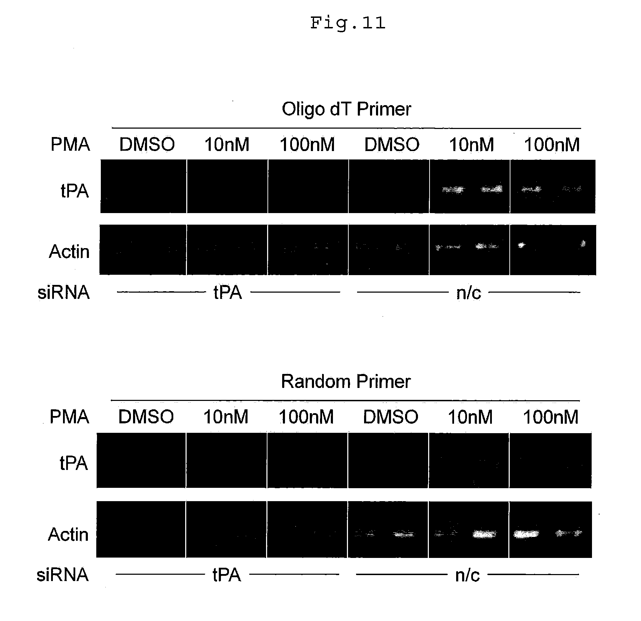

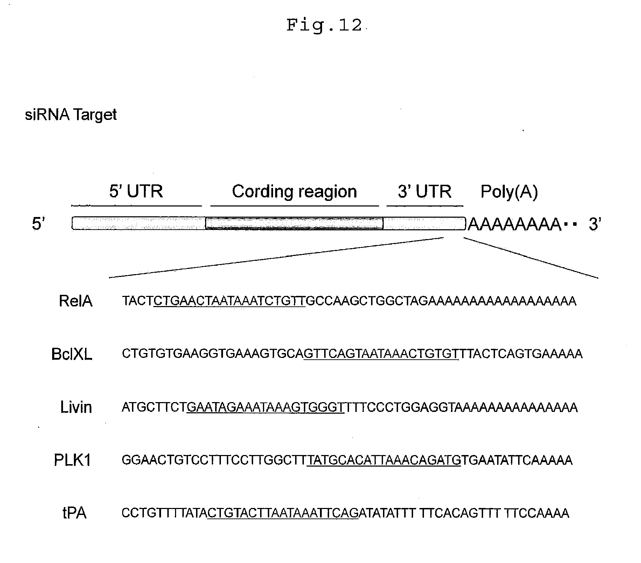

There were designed siRNAs that would cover the polyadenylation signal sequences at 3'-UTR of respective mRNAs of *RelA, Bcl-xL, Livin* and *PLK1* (Fig. 12). When the siRNAs were transferred into HeLa cells, both mRNA and protein accumulations were found to decrease specifically (Figs. 8 to 10). Further, with respect to *tPA* gene whose expression is induced by PMA addition, siRNA was also designed that would cover the polyadenylation signal sequence at 3'-UTR of tPA mRNA (Fig. 12). The siRNA was added simultaneously with the addition of PMA to HeLa cells. Twenty-four hours later, RNA was recovered. RT-PCR analysis revealed that induction of tPA expression was inhibited at the mRNA level (Fig. 11).

### Discussion

This experiment provides siRNAs which can be designed more simply than in conventional methods.

All publications, patents and patent applications cited herein are incorporated herein by reference with their entirety.

### INDUSTRIAL APPLICABILITY

The present invention is applicable as a method of selective gene downregulation.

### SEQUENCE LISTING FREE TEXT

<SEQ ID NO: 1>
   SEQ ID NO: 1 shows the nucleotide sequence of antisense morpholino oligonucleotide cdk9 MO.
<SEQ ID NO: 2>
   SEQ ID NO: 2 shows the nucleotide sequence of antisense morpholino oligonucleotide cdk9 MO-2.
<SEQ ID NO: 3>
   SEQ ID NO: 3 shows the nucleotide sequence of antisense morpholino oligonucleotide cdk9 MO-3.
<SEQ ID NO: 4>
   SEQ ID NO: 4 shows the nucleotide sequence of antisense morpholino oligonucleotide cdk9 MO-4.
<SEQ ID NO: 5>
   SEQ ID NO: 5 shows the nucleotide sequence of antisense morpholino oligonucleotide cdk9 MO-5.
<SEQ ID NO: 6>
   SEQ ID NO: 6 shows the nucleotide sequence of antisense morpholino oligonucleotide cdk9 MO-6.
<SEQ ID NO: 7>
   SEQ ID NO: 7 shows the nucleotide sequence of antisense morpholino oligonucleotide cdk9 MO-7.
<SEQ ID NO: 8>
   SEQ ID NO: 8 shows the nucleotide sequence of antisense morpholino oligonucleotide cdk9 MO-8.
<SEQ ID NO: 9>
   SEQ ID NO: 9 shows the nucleotide sequence of antisense morpholino oligonucleotide cdk9m MO.
<SEQ ID NO: 10>
   SEQ ID NO: 10 shows the nucleotide sequence of (dT)₁₂-anchor primer.
<SEQ ID NO: 11>
   SEQ ID NO: 11 shows the nucleotide sequence of PAT assay primer zcdk9 PAT.
<SEQ ID NO: 12>
   SEQ ID NO: 12 shows the nucleotide sequence of PAT assay primer tbp PAT.
<SEQ ID NO: 13>
   SEQ ID NO: 13 shows the nucleotide sequence of PAT assay primer cyclin B1 PAT.
<SEQ ID NO: 14>
   SEQ ID NO: 14 shows the nucleotide sequence of PAT assay primer cyclin B2 PAT.
<SEQ ID NO: 15>
   SEQ ID NO: 15 shows the nucleotide sequence of actin-specific primer (forward) for PCR.
<SEQ ID NO: 16>
   SEQ ID NO: 16 shows the nucleotide sequence of actin-specific primer (reverse) for PCR.
<SEQ ID NO: 17>
   SEQ ID NO: 17 shows the nucleotide sequence of biklf-specific primer (forward) for PCR.
<SEQ ID NO: 18>
   SEQ ID NO: 18 shows the nucleotide sequence of biklf-specific primer (reverse) for PCR.
<SEQ ID NO: 19>
   SEQ ID NO: 19 shows the nucleotide sequence of zcdk9-specific primer (forward) for PCR.
<SEQ ID NO: 20>
   SEQ ID NO: 20 shows the nucleotide sequence of zcdk9-specific primer (reverse) for PCR.
<SEQ ID NO: 21>
   SEQ ID NO: 21 shows the nucleotide sequence of tbp-specific primer (forward) for PCR.
<SEQ ID NO: 22>
   SEQ ID NO: 22 shows the nucleotide sequence of tbp-specific primer (reverse) for PCR.
<SEQ ID NO: 23>
   SEQ ID NO: 23 shows the nucleotide sequence of cyclin B1-specific primer (forward) for PCR.
<SEQ ID NO: 24>
   SEQ ID NO: 24 shows the nucleotide sequence of cyclin B1-specific primer (reverse) for PCR.
<SEQ ID NO: 25>
   SEQ ID NO: 25 shows the nucleotide sequence of cyclin B2-specific primer (forward) for PCR.
<SEQ ID NO: 26>
   SEQ ID NO: 26 shows the nucleotide sequence of cyclin B2-specific primer (reverse) for PCR.
<SEQ ID NO: 27>
   SEQ ID NO: 27 shows the nucleotide sequence of siRNA against RelA.
<SEQ ID NO: 28>
   SEQ ID NO: 28 shows the nucleotide sequence of siRNA against Bcl-xL.
<SEQ ID NO: 29>
   SEQ ID NO: 29 shows the nucleotide sequence of siRNA against Livin.
<SEQ ID NO: 30>
   SEQ ID NO: 30 shows the nucleotide sequence of siRNA against PLK1.
<SEQ ID NO: 31>
   SEQ ID NO: 31 shows the nucleotide sequence of siRNA against tPA.
<SEQ ID NO: 32>
   SEQ ID NO: 32 shows the nucleotide sequence of RelA-specific primer (forward).
<SEQ ID NO: 33>
   SEQ ID NO: 33 shows the nucleotide sequence of RelA-specific primer (reverse).
<SEQ ID NO: 34>
   SEQ ID NO: 34 shows the nucleotide sequence of Bcl-xL-specific primer (forward).
<SEQ ID NO: 35>
   SEQ ID NO: 35 shows the nucleotide sequence of Bcl-xL-specific primer (reverse).
<SEQ ID NO: 36>
   SEQ ID NO: 36 shows the nucleotide sequence of PLK1-specific primer (forward).
<SEQ ID NO: 37>
   SEQ ID NO: 37 shows the nucleotide sequence of PLK1-specific primer (reverse).
<SEQ ID NO: 38>
   SEQ ID NO: 38 shows the nucleotide sequence of tPA-specific primer (forward).
<SEQ ID NO: 39>
   SEQ ID NO: 39 shows the nucleotide sequence of tPA-specific primer (reverse).
<SEQ ID NO: 40>
   SEQ ID NO: 40 shows the nucleotide sequence of Livin-specific primer (forward).
<SEQ ID NO: 41>
   SEQ ID NO: 41 shows the nucleotide sequence of Livin-specific primer (reverse).
<SEQ ID NO: 42>
   SEQ ID NO: 42 shows the nucleotide sequence of β actin-specific primer (forward).
<SEQ ID NO: 43>
   SEQ ID NO: 43 shows the nucleotide sequence of β actin -specific primer (reverse).

## Claims

1. A method of inhibiting the translation reaction of a target gene, comprising cutting out a part of the poly(A) tail and/or 3'-terminal sequence of the target mRNA.

2. The method according to claim 1, wherein a part of the poly(A) tail and/or 3'-terminal sequence of the target mRNA is cut out with a ribonucleic acid which is capable of hybridizing to the part of the poly(A) tail and/or 3'-terminal sequence of the target mRNA.

3. The method according to claim 2, wherein the ribonucleic acid comprises a nucleotide sequence complementary to the whole or a part of a sequence encoding the region from the poly(A) tail junction of the target mRNA to the 40^{th} nucleotide upstream therefrom.

4. The method according to claim 3, wherein the whole or a part of a sequence encoding the region of 40 nucleotides from the poly(A) tail junction of the target mRNA to the 40^{th} nucleotide upstream therefrom contains the whole or a part of a polyadenylation signal sequence.

5. The method according to claim 3 or 4, wherein the ribonucleic acid is a 20- to 25-mer.

6. The method according to any one of claims 2 to 5, wherein the ribonucleic acid comprises natural nucleotides.

7. The method according to claim 6, wherein the ribonucleic acid is a double-stranded RNA.

8. The method according to any one of claims 2 to 5, wherein the ribonucleic acid contains at least one nucleotide analogue.

9. The method according to claim 8, wherein the ribonucleic acid is single-stranded.

10. The method according to claim 9, wherein the single-stranded ribonucleic acid is an antisense morpholino oligonucleotide.

11. A kit for inhibiting the translation reaction of a target gene, comprising a reagent capable of cutting out a part of the poly(A) tail and/or 3'-terminal sequence of the target mRNA.

12. The kit according to claim 11, wherein the reagent capable of cutting out a part of the poly(A) tail and/or 3'-terminal sequence of the target mRNA is a ribonucleic acid.

13. A cell in which the translation reaction of a target gene is inhibited by introduction thereinto of a reagent capable of cutting out a part of the poly(A) tail and/or 3'-terminal sequence of the target mRNA.

14. A non-human organism in which the translation reaction of a target gene is inhibited by introduction thereinto of a reagent capable of cutting out a part of the poly(A) tail and/or 3'-terminal sequence of the target mRNA.
